# EUROPÄISCHE PATENTSCHRIFT

(11) **EP 1 831 680 B1**
(45) Veröffentlichungstag und Bekanntmachung des Hinweises auf die Patenterteilung: **17.12.2014**
(21) Anmeldenummer: 05795923.1
(22) Anmeldetag: 21.10.2005
(51) Int. Cl.: G01N 30/12, B01D 53/04, G01N 33/00, G01N 1/40

(54) **Verfahren zur Elementaranalyse einer organischen Probe, die durch Verbrennung aufgeschlossen wird, und Vorrichtung zur Durchführung des Verfahrens**
Method for the elementary analysis of an organic sample digested via combustion, and corresponding apparatus
Procédé d'analyse élémentaire d'un échantillon organique libéré par combustion, et dispositif correspondant

(30) Priorität: 27.12.2004 DE 102004063613
(43) Veröffentlichungstag der Anmeldung: 12.09.2007
(73) Patentinhaber: Elementar Analysensysteme GmbH, 63452 Hanau (DE)
(72) Erfinder: KUPKA, Hans-Joachim, 63543 Neuberg (DE); SIEPER, Hans-Peter, 63471 Gelnhausen (DE); WEIGAND, Walter, 63579 Freigericht (DE)
(74) Vertreter: Grimm, Ekkehard
(86) Internationale Anmeldenummer: PCT/EP2005/011337
(87) Internationale Veröffentlichungsnummer: WO 2006/074720

(56) Entgegenhaltungen:
- EP-A- 1 536 452
- DE-A1- 4 231 509
- DE-A1- 10 035 409
- US-A- 5 142 143
- PATENT ABSTRACTS OF JAPAN Bd. 012, Nr. 062 (P-670), 25. Februar 1988 (1988-02-25) -& JP 62 204157 A (MITSUI TOATSU CHEM INC), 8. September 1987 (1987-09-08)
- HROUZKOVA S ET AL: "Carbon sorbents as a mean for enrichment of atmospheric oxygenated VOCs with subsequent HRGC determination" INT J ENVIRON ANAL CHEM; INTERNATIONAL JOURNAL OF ENVIRONMENTAL ANALYTICAL CHEMISTRY, GORDON & BREACH SCIENCE PUBL INC., NEWARK, NJ, USA, Bd. 74, Nr. 1, 2. März 1998 (1998-03-02), Seiten 91-106, XP009061639

## Beschreibung

Die Erfindung betrifft ein Verfahren zur Elementaranalyse einer organischen Probe, die durch Verbrennung aufgeschlossen wird, wobei die gasförmigen Verbrennungsprodukte mittels eines inerten Trägergases eines Hauptgasstroms einem adsorbierenden Material zugeführt werden und mindestens zwei Gase im Trägergas durch das adsorbierende Material geführt werden und die zu analysierenden/trennenden Gase dort adsorbiert und anschließend durch Aufheizen desorbiert werden, wonach die einzelnen Gaskomponenten einer Analyse- und/oder einer Auffangvorrichtung zugeführt werden, sowie eine Vorrichtung zur Durchführung des Verfahrens.

Ein derartiges Verfahren ist in der DE 42 31 509 A1 beschrieben. Dieses Dokument gibt ein Verfahren zur Analyse einer organischen Probe an. Die in dem Dokument beschriebene Vorrichtung zur Bestimmung des Gehalts an Wasserstoff, Kohlenstoff, Stickstoff und Schwefel in einer organischen Probe weist einen Probenverbrennungsteil auf, in dem die Probe in einem Helium-Trägergasstrom unter Zusatz von Sauerstoff in die Komponenten Wasserdampf, Kohlendioxid, Stickstoff und Schwefeldioxid aufgeschlossen wird. Diese Komponenten werden in dem Trägergasstrom durch eine Wasserdampf-Adsorptionsfalle geleitet, wo Wasserdampf adsorbiert wird. Die anderen Komponenten, die hindurchgelassen werden, werden in einer Schwefeldioxid-Adsorptionsfalle geleitet, das Schwefeldioxid adsorbiert, allerdings die beiden anderen Komponenten hindurchlässt. Der Schwefeldioxid-Adsorptionsfalle ist eine Kohlendioxid-Adsorptionsfalle nachgeordnet, in der Kohlendioxid, allerdings nicht Stickstoff, zurückgehalten wird. Bei dieser Vorrichtung sind somit drei einzelne Adsorptionsfallen, die der Verbrennungseinrichtung nachgeordnet sind, vorhanden, wodurch sich ein umfangreicher, apparativer Aufbau ergibt.

Die DE 100 35 409 A1 beschreibt eine differentielle Thermodesorption für Gassensorsysteme. Bei diesem System ist zwischen der Analytquelle und dem Sensorsystem ein Thermodesorptionsröhrchen angeordnet. Dadurch kann die Analytgasmischung angereichert werden (Adsorption) und dann unter Beibehaltung der Flussrichtung und mittels einer kontrollierten Temperaturrampe vollständig oder teilweise getrennt (chromatografischer Effekt) wieder freigesetzt werden (Desorption). Wie sich aus den Unterlagen weiterhin ergibt, beruht das System auf der Kopplung mehrerer teilspezifischer Sensoren in Kombination mit dem Thermodesorptionsröhrchen. Die Thermodesorptionsröhrchen werden in erster Linie für die notwendige Anreicherung der (unter Umständen unbekannten) Proben genutzt, dann wird schrittweise geheizt und damit Analyt für Analyt den Sensoren zugeführt.

Die US-A-5 142 143 beschreibt ein Verfahren und eine Vorrichtung zum Erfassen von Spurenbestandteilen in Luft oder anderen Gasen, insbesondere zum Vorkonzentrieren dieser Bestandteile, um eine empfindlichere Erfassungsmessung zu erreichen. Das Verfahren umfasst ein Adsorbieren mindestens eines Spurenbestandteils auf einem eingegrenzten Sorptionsmaterial (sorbent) für eine vorgegebene Zeitdauer von einem Probengas, das sich unter einer vorgegebenen Gasmassenflussrate bewegt, Einbringen des Sorptionsmaterials in einen abgedichteten Raum, der einen Einlass für eine Detektoreinrichtung umfasst, und Desorbieren des einen Spurenelements von dem Sorptionsmaterial.

PATENT ABSTRACTS OF JAPAN, Bd. 012, Nr. 062 (P-670), 25. Februar 1988; - & JP 62 204157 A (MITSUI TOATSU CHEM INC), 8. September 1987, beschreibt ein Verfahren für eine einfache, quantitave Analyse von niedrigen Konzentrationen an Phosphin. Hierzu wird eine Gasmischung durch ein Adsorptionsröhrchen geleitet und das Phosphin selektiv angereichert. Die Methode dient nicht in erster Linie zur Gastrennung, da anschließend an die Desorption noch eine gaschromatografische Trennung mit konventioneller Säule angeschlossen ist.

Das Dokument HROUZKOVA S ET AL: "Carbon sorbents as a mean for enrichment of atmospheric oxygenated VOCs with subsequent HRGC determination", INT J ENVIRON ANAL CHEM; INTERNATIONAL JOURNAL OF ENVIRONMENTAL ANALYTICAL CHEMISTRY, GORDON & BREACH SCIENCE PUBL. INC:, NEWARK, NJ, USA, Bd. 74, Nr. 1, 2. März 1998, Seiten 91-106, XP009061639, behandelt die Evaluierung einer Probenvorkonzentration mit einem speziellen experimentellen Aufbau, der kleinste Mengen organischer Stoffe aus der Atmosphäre anreichern kann. Die eigentliche Gastrennung und anschließende Quantifizierung finden in einem herkömmlichen Gaschromatografen mit Kapillarsäule statt. Eine Erweiterung des Konzentrationsbereiches bis in den Milligramm-Bereich wird bei diesem System nicht erreicht; es handelt sich um Spurengasmessungen.

Durch die Elementaranalyse, wie sie Gegenstand der vorliegenden Erfindung ist und wie sie auch in dem vorstehend angegebenen Dokument erläutert ist, werden Elemente, die in organischen Verbindungen enthalten sind, wie Kohlenstoff und Wasserstoff, Sauerstoff, Stickstoff, Schwefel, Halogene, sowohl qualitativ als auch quantitativ bestimmt. Wesentlich ist, dass relativ große Probenmengen analysiert werden können, die im Bereich von 2 g für feste Proben und im Bereich von 5 ml für flüssige Proben liegen können.

Von dem vorstehend beschriebenen Verfahren der Gastrennung für die Elementaranalyse ist die Gaschromatografie zu unterscheiden. Dabei werden die Gasgemische mit einem Trägergas im gleichmäßigen Strom durch eine Säule geleitet, in der sich ein Adsorptionsmaterial als stationäre Phase befindet. Durch unterschiedliche Wechselwirkungen der Gaskomponenten mit dem Adsorptionsmaterial erfolgt eine zeitliche und räumliche Trennung im Gasstrom. Im nachfolgenden Detektor können die Komponenten als Peaks quantitativ bestimmt werden. Die Gaschromatografie stellt ein sehr leistungsfähiges Verfahren zur Analyse auch von sehr komplexen Stoffgemischen dar. Die Probenmenge ist allerdings auf einige µl begrenzt und die Geschwindigkeit des Trägergasstroms ist auf etwa 10 bis 20 ml/min begrenzt. Die Absolutmengen der analysierbaren Elemente bzw. ihrer Verbrennungsgase sind auf wenige Milligramm eingeschränkt. Weiterhin ist apparativ eine hohe Temperaturkonstanz (± 1°C) der Adsorptionstrennsäule erforderlich.

Der vorliegenden Erfindung liegt die Aufgabe zugrunde, ein Verfahren der eingangs genannten Art anzugeben, das gegenüber bisherigen Verfahren zur Elementaranalyse vereinfachter durchzuführen ist, insbesondere auch dann, wenn mehr als zwei Gase analysiert oder getrennt werden sollen; weiterhin soll eine entsprechende Vorrichtung angegeben werden, die sich durch ihren gegenüber herkömmlichen Vorrichtungen einfacheren Aufbau auszeichnet.

Gegenüber der gaschromatografischen Gastrennung soll weiterhin die Möglichkeit bestehen, mit geringerem apparativen Aufwand Elemente in einem größeren absoluten Konzentrationsbereich sowie Proben mit höherer Einwaage zu bestimmen.

Gelöst wird diese Aufgabe erfindungsgemäß durch ein Verfahren zur Elementaranalyse einer organischen Probe mit den Merkmalen des Anspruchs 1 sowie eine Vorrichtung nach Anspruch 15.

Vorteilhafte Ausführungsformen ergeben sich aus den abhängigen Ansprüchen.

Mit dem erfindungsgemäßen Verfahren können somit mindestens zwei Gase im Trägergas eines Hauptgasstroms analysiert werden oder aber auch voneinander getrennt werden, ohne dass hierzu mehrere Adsorptionssäulen, wie im vorstehend beschriebenen Stand der Technik, die Elementaranalyse betreffend, erforderlich sind. Vielmehr wird eine Adsorptionssäule der Verbrennungseinrichtung nachgeordnet, die dann in Folge mit einer entsprechenden Heizeinrichtung auf die Temperaturen aufgeheizt wird, die für die Desorption der jeweils zu bestimmenden Elemente spezifisch sind. Die jeweiligen spezifischen Temperaturen werden für eine bestimmte Zeitdauer gehalten, bevor dann auf die jeweils nächste, höhere Temperatur, die für den zu bestimmenden Bestandteil spezifisch ist, aufgeheizt wird. Es ist auch möglich, die Temperatur mit einer konstanten Rate zu erhöhen. Dabei werden die spezifischen Freisetzungstemperaturen für die jeweilige Gasart in einer bestimmten Zeit durchlaufen. Das Aufheizen in Form einer Treppenstufenfunktion, d.h. das Halten der einzelnen, spezifischen Temperaturen, ist allerdings in den meisten Fällen bevorzugt, um eine bessere Trennung der einzelnen Peaks zu erhalten. Zur Elementaranalyse werden die jeweiligen unter der spezifischen Temperatur freigesetzten Bestandteile einer dafür geeigneten Detektionsvorrichtung zugeführt oder sie werden, zur präparativen Trennung von zwei im Trägergas enthaltenen Bestandteilen, die bei den jeweiligen spezifischen Temperaturen freigesetzt werden, einer geeigneten Auffangvorrichtung (z. B. einer Kältefalle) zugeführt. Im Vergleich zur bekannten Vorrichtung, bei der die Gaskomponententrennung vorzugsweise durch verschiedene komponentenspezifische Adsorptionsmaterialien in verschiedenen Adsorptionssäulen erfolgt, kommt beim erfindungsgemäβen Verfahren lediglich eine Adsorptionssäule zum Einsatz. Die Gastrennung erfolgt dann vorzugsweise durch verschiedene komponentenspezifische Desorptionstemperaturen. Gute Ergebnisse haben das erfindungsgemäße Verfahren sowie die entsprechende Vorrichtung bei Probenanalysen gezeigt; die Ergebnisse sind reproduzierbar und zeigen praktisch keine Abweichungen zu Elementaranalyseverfahren, die unter Verwendung der eingangs beschriebenen, bekannten Vorrichtung erreicht werden.

Weitere Vorteile des erfindungsgemäßen Verfahrens sowie der entsprechenden Vorrichtung sind:

Das Verfahren hat neben dem einfachen Aufbau und dem einfachen Betrieb den Vorteil, dass die Temperatur nicht mit hoher Präzision wie bei der Gaschromatografie eingehalten werden muss. Weiterhin ist die Trennung über Temperaturprogramme im Vergleich zur Gaschromatografie in geringerem Maße von der Konstanz des Trägergasflusses abhängig. Die Adsorption des gesamten Gasgemisches und die schnelle Freisetzung der Komponenten durch Temperaturerhöhung bewirken weiterhin eine Konzentrationserhöhung im jeweiligen Komponenten-Peak sowie eine größere Konzentrationsdynamik im Vergleich zur Gaschromatografie. Daher ist außer dem erweiterten Einsatz in der Elementaranalyse auch die präparative Gastrennung ein mögliches Anwendungsgebiet.

Ein weiterer Vorteil des erfindungsgemäßen Verfahrens ist ein geringer Zeitbedarf, da die Zeit zwischen zwei getrennten Gaskomponenten kontrolliert minimiert werden kann und Temperaturerhöhung eine Beschleunigung der Desorption bewirkt. Außerdem kann mit höheren Gasflüssen gearbeitet werden.

Das verwendete Prinzip der thermisch gesteuerten Desorption findet traditionell in der Thermischen Desorptionsspektroskopie (TDS) Anwendung. Sie dient in der Grundlagenforschung zur Messung der Kinetik von Adsorption und Desorption von Gasen, wie CO oder H₂ an Metalloberflächen unter Hochvakuumbedingungen (D.P. Woodruff, T.A. Delchov, Modern Techniques of Surface Science - Second Edition, Cambridge University Press 1994, S. 356-377).

Im Bereich der Analysentechnik wird die thermische Gasdesorption als Probenvorbereitungsmethode zur VOC (volatile organic carbon) Analyse eingesetzt. Dabei wird z.B. eine Luftprobe über eine Adsorptionssäule gesaugt, auf der enthaltene organische Verbindungen gesammelt werden. Im Labor wird der VOC durch Aufheizen wieder freigesetzt und mit einem Trägergas dem Analysator (Gaschromatograf oder Massenspektrometer) zugeführt.

Im Unterschied zum erfindungsgemäßen Verfahren wird die thermische Desorption nicht zur Gastrennung, sondern lediglich Sammlung bzw. Vorkonzentrierung, verwendet. Auβerdem findet sie keine Anwendung auf anorganische Gase, wie z.B. SO₂, CO₂ oder H₂O, die für die Elementaranalyse Bedeutung haben.

Bevorzugt wird als adsorbierendes Material ein Molekularsieb eingesetzt; sehr gute Ergebnisse wurden mit einem Kohlenstoff-Molekularsieb als Adsorptionsmaterial erreicht. Dies gilt insbesondere dann, wenn mit dem Verfahren der Gehalt an N₂, CO₂, H₂O und SO₂ einer Probe bestimmt werden soll. Speziell wurden Kohlenstoff-Molekularsiebe herangezogen, die eine große, spezifische Oberfläche zeigen, und zwar von 1000 m²/g und darüber. Solche Kohlenstoff-Molekularsiebe werden von der Firma Supelco unter der Bezeichnung Carboxen-1000 und Carboxen-1003 vertrieben.

Für die Desorption von N₂-Gas wird eine Desorptionstemperatur im Bereich von Raumtemperatur bis etwa 50°C eingestellt. Die Dauer, für die diese Desorptionstemperatur gehalten wird, richtet sich nach der Probenmenge; es reichen etwa 60 Sekunden aus.

Für die Desorption von CO₂-Gas wird eine Desorptionstemperatur im Bereich von 50°C bis 70°C, vorzugsweise bei etwa 60°, eingestellt. Die Dauer, für die die ausgewählte Temperatur beibehalten wird, sollte etwa zwischen 100 und 180 Sekunden betragen.

Für die Desorption von H₂O wird eine Desorptionstemperatur im Bereich von 140°C bis 160°C, vorzugsweise bei etwa 150°, eingestellt; eine Zeitdauer von 60 Sekunden, für die diese Temperatur konstant gehalten wird, reicht aus, um H₂O zu desorbieren.

Für die Desorption von SO₂-Gas wird eine Desorptionstemperatur im Bereich von 200°C bis 220°C, vorzugsweise von etwa 210°, eingestellt. Die eingestellte Temperatur sollte vorzugsweise etwa für 60 Sekunden beibehalten werden.

Es sollte verständlich werden, dass die gesamte Dauer der Temperaturbehandlung bei den einzelnen Temperaturen für die Desorption von CO₂-Gas, H₂O und SO₂-Gas zwischen 6 und 10 Minuten, vorzugsweise 8 Minuten oder geringer, betragen kann, um eine Analyse abzuschließen.

Die Temperaturmessungen können direkt an der Heizung vorgenommen werden.

Zwischen einzelnen Probemessungen wird die Anordnung auf Raumtemperatur zwangsgekühlt, d.h. die Anordnung wird wieder abgekühlt, bevor die Analyse der nächsten Probe, und unter Verbrennung der entsprechenden Probemenge, erfolgt.

Der Gasvolumenstrom sollte bei 200 ml/min bis 700 ml/min liegen.

Zur Analyse einer organischen Probe wird die Probe durch Verbrennung aufgeschlossen und die gasförmigen Verbrennungsprodukte werden mittels eines inerten Trägergases dem adsorbierenden Material zugeführt.

Mit dem angegebenen Verfahren können Elementmengen an N, C, H und S von über 10 mg getrennt und analysiert werden.

Die jeweiligen Änderungen zwischen den Desorptionstemperaturen erfolgen vorzugsweise als Treppenfunktion, d.h. die einzelnen Temperaturen werden unmittelbar auf den jeweiligen Sollwert geändert.

Vorrichtungsgemäß wird die Adsorptionssäule durch ein Rohr mit guten Wärmeleiteigenschaften und korrosionsbeständige Oberfläche, beispielsweise aus Edelstahl, gebildet.

Das Adsorptionsrohr sollte eine Länge von etwa 250 mm bis 350 mm, vorzugsweise von 300 mm, aufweisen. Der Innendurchmesser eines solchen Adsorptionsrohrs liegt bei 4 mm bis 10 mm, vorzugsweise bei etwa 6 mm.

Für einen kompakten Aufbau wird das Adsorptionsrohr U-förmig gebogen.

Um zwischen einzelnen Probenmessungen wieder dieselben Ausgangsbedingungen zu erhalten, ist dem Adsorptionsrohr eine Zwangskühleinrichtung zugeordnet, mit der das Adsorptionsrohr nach Abschluss der Messungen schnell wieder auf Raumtemperatur abgekühlt werden kann. Eine solche Zwangskühleinrichtung kann durch ein Luftgebläse erfolgen.

Für die Temperaturmessung kann an der Heizeinrichtung ein Thermoelement angebracht werden.

Nachfolgend werden ein Ausführungsbeispiel der Vorrichtung sowie ein Verfahren für die Elementaranalyse unter Bezugnahme auf die Zeichnungen beschrieben. In der Zeichnung zeigt
- Figur 1: ein Diagramm mit der Temperaturmesskurve und den jeweiligen Desorptions-Peaks für N₂, CO₂, H₂O und SO₂, und
- Figur 2: ein Adsorptionsrohr, das für die in Figur 1 gezeigte Messung eingesetzt wurde.

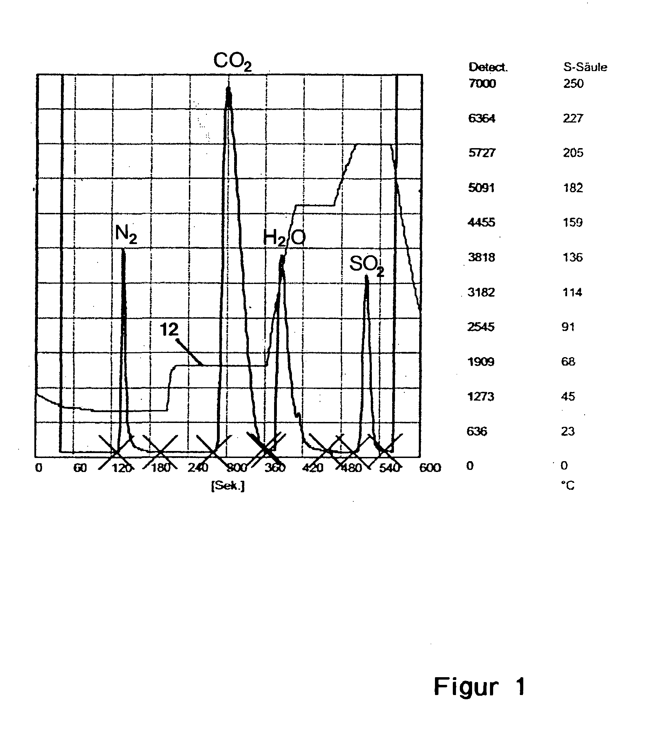

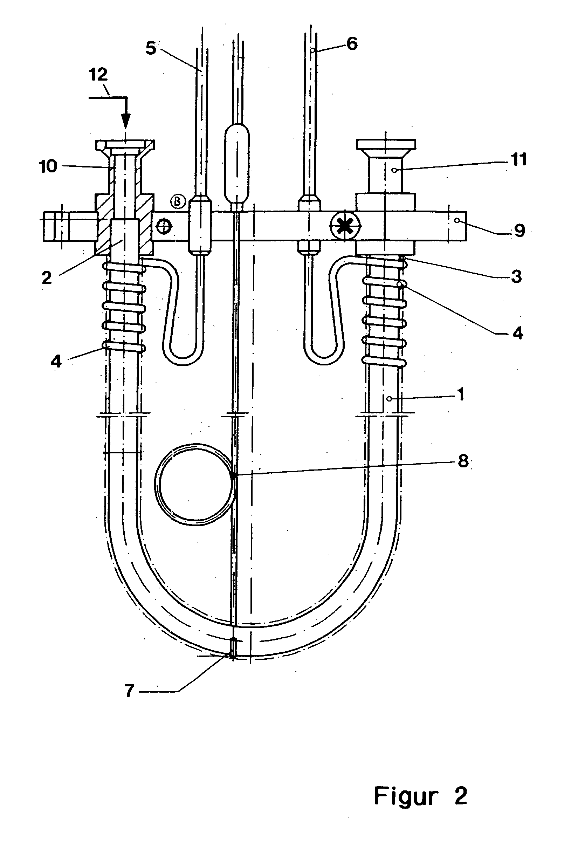

Das Diagramm, wie es in Figur 1 gezeigt ist, stellt die Elementaranalyse von N₂, CO₂, H₂O und SO₂ dar, wobei die Adsorptionssäule eingesetzt wurde, wie sie in Figur 2 dargestellt ist.

Die Adsorptionssäule, wie sie in einer Seitenansicht in Figur 2 zu sehen ist, umfasst ein U-förmiges Rohr 1 mit einer Länge entlang dessen Achse, zwischen den beiden Enden 2, 3, von etwa 300 Millimetern. Der Innendurchmesser des Adsorptionsrohrs 1 liegt bei etwa 6 mm. Die Außenseite des U-förmigen Adsorptionsrohrs 1 ist von einer spiralförmig gewikkeiten Heizwicklung 4 umgeben, mit Versorgungsleitungen 5 und 6 am oberen Ende. Diese Heizwicklung 4 ist, obwohl sie nur im oberen Bereich des U-förmigen Rohrs 1 gezeichnet ist, entlang des gesamten Rohrs vorhanden. Im unteren Bogen des U-förmigen Rohrs 1 ist der Heizwicklung 4, auf der Außenseite des Rohrs 1, ein Thermoelement 7 zugeordnet, das über Leitungen 8 mit einer nicht näher gezeigten Temperaturmesseinrichtung verbunden ist. Über dieses Thermoelement 7 kann die jeweilige Ist-Temperatur an der Heizwicklung 4 im unteren Bereich des Rohrs 1 erfasst werden und mit der jeweiligen SollTemperatur abgeglichen werden.

Die Enden 2, 3 des U-förmigen Rohrs 1 sind in einem Haltebügel 9 eingeklemmt und weisen jeweils einen Anschlussstutzen 10 und 11 auf. Das U-förmige Rohr 1 ist mit einem adsorbierenden Material in Form eines Kohlenstoff-Molekularsiebs gefüllt. Der in Figur 2 linke Anschlussstutzen 10 ist mit einer Verbrennungseinrichtung, die nicht dargestellt ist, verbunden. In dieser Verbrennungseinrichtung wird eine zu analysierende Probe verbrannt und die Verbrennungsprodukte werden über einen Trägergasstrom der Adsorptionssäule zugeführt, was durch den Pfeil 12 angedeutet ist. Die in dem Trägergasstrom mitgeführten Bestandteile an Wasserdampf, Kohlendioxid, Stickstoff und Schwefeldioxid werden an dem Kohlenstoff-Molekularsieb adsorbiert. Diese Adsorption erfolgt in einem Temperaturbereich zwischen Raumtemperatur und ca. 45° - 50°C, innerhalb der Adsorptionssäule der Figur 2.

Anschließend wird die Adsorptionssäule über die Heizwicklung 4 auf spezifische Temperaturen aufgeheizt, um die Bestandteile N₂, CO₂, H₂O und SO₂ zu desorbieren.

Unter den jeweiligen spezifischen Temperaturen werden dann über ca. 45°C / ca. 60°C / ca. 150°C / ca. 210°C die Anteile an N₂, CO₂, H₂O und SO₂ erfasst.
In Figur 1 ist ein entsprechendes Messdiagramm über eine Messdauer von 600 Sekunden dargestellt. Die über die Heizwicklung 4 eingestellte und über das Thermoelement 7 gemessene Temperatur ist in Form der Messkurve 12 in Figur 1 gezeigt, wobei die absoluten Temperaturwerte in der rechten Spalte anhand der angegebenen Zahlenwerte in Grad Celsius abzulesen sind. In der Spalte links neben den Temperaturangaben sind Detektionswerte, in absoluten Einheiten, angegeben, die den jeweiligen Peaks zugeordnet werden können.

Zunächst wurde das Adsorptionsrohr 1 der Figur 2 auf 40°C, für eine Messdauer von 180 Sekunden, gehalten. Nach etwa 180 Sekunden konnte ein Peak, der N₂ zuzuordnen ist, gemessen werden. Nach Desorption des vorhandenen Stickstoffs wurde die Temperatur auf 60°C erhöht und dann für etwa 150 Sekunden konstant gehalten. Die Desorption von CO₂ stellte sich nach etwa 280 Sekunden seit Messbeginn (Nullpunkt in dem Diagramm der Figur 1) ein. Danach, d.h. nach 360 Sekunden seit Messbeginn, wurde die Temperatur auf 150°C erhöht und für 60 Sekunden gehalten. Bereits mit der Temperaturerhöhung wurde H₂O, d.h. Wasserdampf, desorbiert, und konnte als entsprechender Peak, der in Figur 1 mit H₂O gekennzeichnet ist, gemessen werden. Schließlich wurde die Temperatur auf etwa 210°C erhöht und wiederum für etwa 60 Sekunden gehalten. Bei dieser Temperatur konnte ein der Desorption von SO₂ zuzuordnender Peak gemessen werden.

Nach der Trennung erfolgt der Nachweis der Komponenten im Trägergas, z.B. mit einem Wärmeleitfähigkeitsdetektor. Es können aber auch andere (z.B. spektrofotometrische) Detektionsprinzipien zum Einsatz kommen.

Anhand der grafischen Darstellung der Figur 1 ist zu erkennen, dass, obwohl nur eine Adsorptionssäule eingesetzt wurde, deutlich die jeweiligen Desorptions-Peaks für N₂, CO₂, H₂O und SO₂ unterschieden werden können. Solche Messungen sind reproduzierbar und es wurde bestätigt, dass sie vergleichbare Werte erzielen, wie sie mit aufwendigeren Anordnungen nach dem Stand der Technik möglich sind.

In dem konkreten Fall, wie er in Figur 1 dargestellt ist, wurde das Probenmaterial Sulfanilamid, Probenmenge ca. 5 mg, in der Verbrennungseinrichtung gemessen.

## Patentansprüche

1. Verfahren zur Elementaranalyse einer organischen Probe, die durch Verbrennung aufgeschlossen wird, wobei die gasförmigen Verbrennungsprodukte mittels eines inerten Trägergases eines Hauptgasstroms einem adsorbierenden Material zugeführt werden, wobei mindestens zwei der gasförmigen Verbrennungsprodukte im Trägergas durch das adsorbierende Material geführt werden und die zu analysierenden/trennenden Gase dort adsorbiert und anschließend durch Aufheizen desorbiert werden, wonach die einzelnen Gaskomponenten einer Analyse- und/oder einer Auffangvorrichtung zugeführt werden,
**dadurch gekennzeichnet,**
**dass** eine einzelne Adsorptionssäule eingesetzt wird, die mit einem auf die mindestens zwei Gase abgestimmten, adsorbierenden Material gefüllt ist und die auf ihrer Außenseite direkt beheizt wird, und
**dass** die Temperatur in zeitlicher Folge auf Desorptionstemperaturen, spezifisch für die mindestens zwei Gase, eingestellt wird,
wobei für die Desorption von CO₂-Gas eine Desorptionstemperatur im Bereich von 50°C bis 70°C eingestellt wird,
wobei für die Desorption von N₂-Gas eine Desorptionstemperatur im Bereich von Raumtemperatur bis etwa 50°C eingestellt wird,
wobei für die Desorption von H₂O eine Desorptionstemperatur im Bereich von 140°C bis 160°C eingestellt wird, und
wobei für die Desorption von SO₂-Gas eine Desorptionstemperatur im Bereich von 200°C bis 220°C eingestellt wird.

2. Verfahren nach Anspruch 1, **dadurch gekennzeichnet, dass** als adsorbierendes Material ein Molekularsieb eingesetzt wird.

3. Verfahren nach Anspruch 1 oder 2, **dadurch gekennzeichnet, dass** die Desorption von N₂-Gas bei einer Desorptionstemperatur, die der Raumtemperatur entspricht, durchgeführt wird.

4. Verfahren nach einem der Ansprüche 1 bis 3, **dadurch gekennzeichnet, dass** die Desorption von CO₂-Gas bei einer Desorptionstemperatur von etwa 60°C durchgeführt wird.

5. Verfahren nach einem der Ansprüche 1 bis 4, **dadurch gekennzeichnet, dass** die Desorption von H₂O bei einer Desorptionstemperatur von etwa 150°C durchgeführt wird.

6. Verfahren nach einem der Ansprüche 1 bis 5, **dadurch gekennzeichnet, dass** die Desorption von SO₂-Gas bei einer Desorptionstemperatur von etwa 210°C durchgeführt wird.

7. Verfahren nach den Ansprüchen 1 bis 6, **dadurch gekennzeichnet, dass** die gesamte Dauer der Temperaturbehandlung bei den einzelnen Temperaturen für die Desorption von CO₂-Gas, H₂O und SO₂-Gas zwischen 6 und 10 Minuten beträgt.

8. Verfahren nach Anspruch 7, **dadurch gekennzeichnet, dass** die Temperaturbehandlung 6 bis 8 Minuten beträgt.

9. Verfahren nach einem der Ansprüche 1 bis 8, **dadurch gekennzeichnet, dass** die Temperaturmessung direkt an der Heizung vorgenommen wird.

10. Verfahren nach einem der Ansprüche 1 bis 9, **dadurch gekennzeichnet, dass** zwischen einzelnen Messungen einer Probe die Anordnung auf Raumtemperatur zwangsgekühlt wird.

11. Verfahren nach einem der Ansprüche 1 bis 10, **dadurch gekennzeichnet, dass** der Gasvolumenstrom 200 ml/min bis 700 ml/min beträgt.

12. Verfahren nach einem der Ansprüche 1 bis 11, **dadurch gekennzeichnet, dass** Probenmengen von über 10 mg getrennt und analysiert werden können.

13. Verfahren nach einem der Ansprüche 1 bis 12, **dadurch gekennzeichnet, dass** die jeweiligen Änderungen zwischen den Desorptionstemperaturen als Treppenfunktion erfolgt.

14. Verfahren nach einem der Ansprüche 1 bis 12, **dadurch gekennzeichnet, dass** die Änderungen zwischen den Desorptionstemperaturen auch linear durchlaufen werden können.

15. Vorrichtung eingerichtet zur Durchführung des Verfahrens nach einem der Ansprüche 1 bis 14 mit einer über eine Heizeinrichtung beheizbaren einzelnen Adsorptionssäule, die mit einem adsorbierenden Material gefüllt ist, wobei die Adsorptionssäule ein Gaseinlassende zum Zuführen des Hauptgasstroms sowie ein Gasauslassende aufweist.

16. Vorrichtung nach Anspruch 15, **dadurch gekennzeichnet, dass** die Heizeinrichtung direkt auf der Außenseite der Adsorptionssäule angebracht ist.

17. Vorrichtung nach Anspruch 15 oder 16, **dadurch gekennzeichnet, dass** die Adsorptionssäule durch ein Rohr mit guten Wärmeleiteigenschaften und korrosionsbeständige Oberfläche gebildet ist.

18. Vorrichtung nach Anspruch 17, **dadurch gekennzeichnet, dass** das Rohr aus Edelstahl gebildet ist.

19. Vorrichtung nach Anspruch 17, **dadurch gekennzeichnet, dass** das Adsorptionsrohr eine Länge von etwa 250 mm bis 350 mm, vorzugsweise von 300 mm, aufweist.

20. Vorrichtung nach Anspruch 19, **dadurch gekennzeichnet, dass** der Innendurchmesser des Adsorptionsrohrs 4 mm bis 10 mm, vorzugsweise etwa 6 mm, beträgt.

21. Vorrichtung nach einem der Ansprüche 17 bis 20, **dadurch gekennzeichnet, dass** das Adsorptionsrohr U-förmig gebogen ist.

22. Vorrichtung nach einem der Ansprüche 15 bis 21, **dadurch gekennzeichnet, dass** dem Adsorptionsrohr eine Zwangskühleinrichtung zugeordnet ist.

23. Vorrichtung nach Anspruch 22, **dadurch gekennzeichnet, dass** die Zwangskühleinrichtung durch ein Luftgebläse gebildet ist.

24. Vorrichtung nach einem der Ansprüche 15 bis 23, **dadurch gekennzeichnet, dass** die Adsorptionssäule mit einem Kohlenstoff-Molekularsieb als Adsorptionsmaterial gefüllt ist.

25. Vorrichtung nach einem der Ansprüche 15 bis 24, **dadurch gekennzeichnet, dass** an der Heizeinrichtung ein Thermoelement angebracht ist.

## Claims

1. A method for the elementary analysis of an organic sample which is digested by combustion, wherein the gaseous combustion products are delivered by means of an inert carrier gas of a main gas flow to an adsorbing material, wherein at least two of the gaseous combustion products are conducted in the carrier gas through the adsorbing material and the gases to be analyzed/separated are there adsorbed and subsequently desorbed by being heated, whereupon the individual gas components are delivered to an analyzing and/or collecting device,
**characterized in**
**that** an individual adsorption column is used which is filled with an adsorbing material that is adapted to the at least two gases and which is heated directly on the exterior surface thereof, and
**that** the temperature is successively adjusted to desorption temperatures that are specific for the at least two gases,
wherein for the desorption of CO₂ gases a desorption temperature is adjusted in the range of 50°C to 70°C,
wherein for the desorption of N₂ gas a desorption temperature is adjusted in the range of room temperature up to about 50°C,
wherein for the desorption of H₂O a desorption temperature is adjusted in the range of 140°C to 160°C, and
wherein for the desorption of SiO₂ gas a desorption temperature is adjusted in the range of 200°C to 220°C.

2. The method according to claim 1, **characterized in that** a molecular sieve is used as the adsorbing material.

3. The method according to claim 1 or 2, **characterized in that** the desorption of N₂ gas is carried out at a desorption temperature corresponding to the room temperature.

4. The method according to any one of claims 1 to 3, **characterized in that** the desorption of CO₂ gas is carried out at a desorption temperature of about 60°C.

5. The method according to any one of claims 1 to 4, **characterized in that** the desorption of H₂O is carried out at a desorption temperature of about 150°C.

6. The method according to any one of claims 1 to 5, **characterized in that** the desorption of SO₂ gas is carried out at a desorption temperature of about 210°C.

7. The method according to claims 1 to 6, **characterized in that** the whole duration of the temperature treatment at the individual temperatures for the desorption of CO₂ gas, H₂O and SO₂ gas is between 6 and 10 minutes.

8. The method according to claim 7, **characterized in that** the temperature treatment is 6 to 8 minutes.

9. The method according to any one of claims 1 to 8, **characterized in that** the temperature measurement is directly carried out on the heating unit.

10. The method according to any one of claims 1 to 9, **characterized in that** the assembly is force-cooled to room temperature between individual measurements of a sample.

11. The method according to any one of claims 1 to 10, **characterized in that** the gas volume flow is 200 ml/min to 700 ml/min.

12. The method according to any one of claims 1 to 11, **characterized in that** sample amounts of more than 10 mg can be separated and analyzed.

13. The method according to any one of claims 1 to 12, **characterized in that** the respective changes between the desorption temperatures are carried out as a step function.

14. The method according to any one of claims 1 to 12, **characterized in that** the changes between the desorption temperatures can also be passed through linearly.

15. A device configured to perform the method according to any one of claims 1 to 14, comprising an individual adsorption column which can be heated via a heater and which is filled with an adsorbing material, wherein the adsorption column comprises a gas inlet end for delivering the main gas flow and a gas outlet end.

16. The device according to claim 15, **characterized in that** the heater is directly mounted on the exterior surface of the adsorption column.

17. The device according to claim 15 or 16, **characterized in that** the adsorption column is formed by a tube with good heat conduction properties and corrosion-resistant surface.

18. The device according to claim 17, **characterized in that** the tube is made of special steel.

19. The device according to claim 17, **characterized in that** the adsorption tube has a length of about 250 mm to 350 mm, preferably of 300 mm.

20. The device according to claim 19, **characterized in that** the inner diameter of the adsorption tube is 4 mm to 10 mm, preferably about 6 mm.

21. The device according to any one of claims 17 to 20, **characterized in that** the adsorption tube is bent in U-shaped fashion.

22. The device according to any one of claims 15 to 21, **characterized in that** a forced cooling device is assigned to the adsorption tube.

23. The device according to claim 22, **characterized in that** the forced cooling device is formed by an air blower.

24. The device according to any one of claims 15 to 23, **characterized in that** the adsorption column is filled with a carbon molecular sieve as adsorption material.

25. The device according to any one of claims 15 to 24, **characterized in that** a thermocouple is mounted on the heater.

## Revendications

1. Procédé pour l'analyse élémentaire d'un échantillon organique, lequel est désagrégé par combustion, les produits de combustion gazeux étant introduits au moyen d'un gaz porteur inerte d'un courant de gaz principal sur un matériau adsorbant, au moins deux des produits de combustion gazeux dans le gaz porteur étant introduits à travers le matériau adsorbant et les gaz à analyser/se séparant y étant adsorbés et ensuite désorbés par chauffage, sur quoi les constituants gazeux individuels sont introduits dans un dispositif d'analyse et/ou de recueil,
**caractérisé**
**en ce que** l'on utilise une colonne d'adsorption individuelle, laquelle est remplie d'un matériau adsorbant déterminé par rapport aux au moins les deux gaz et est directement chauffée sur son côté externe, et
**en ce que** la température est ajustée chronologiquement aux températures
de désorption, spécifiquement pour les au moins deux gaz,
une température de désorption pour la désorption de CO₂ gazeux étant ajustée dans un domaine de 50°C à 70°C,
une température de désorption pour la désorption de N₂ gazeux étant ajustée dans un domaine de la température ambiante à environ 50°C,
une température de désorption pour la désorption de H₂O étant ajustée dans un domaine de 140°C à 160°C, et
une température de désorption pour la désorption de SO₂ gazeux étant ajustée dans un domaine de 200°C à 220°C.

2. Procédé selon la revendication 1, **caractérisé en ce que** l'on utilise comme matériau adsorbant un tamis moléculaire.

3. Procédé selon la revendication 1 ou 2, **caractérisé en ce que** la désorption de N₂ gazeux est réalisée à une température de désorption qui correspond à la température ambiante.

4. Procédé selon l'une quelconque des revendications 1 à 3, **caractérisé en ce que** la désorption de CO₂ gazeux est réalisée à une température de désorption d'environ 60°C.

5. Procédé selon l'une quelconque des revendications 1 à 4, **caractérisé en ce que** la désorption de H₂O est réalisée à une température de désorption d'environ 150°C.

6. Procédé selon l'une quelconque des revendications 1 à 5, **caractérisé en ce que** la désorption de SO₂ gazeux est réalisée à une température de désorption d'environ 210°C.

7. Procédé selon les revendications 1 à 6, **caractérisé en ce que** la durée totale du traitement de température aux températures individuelles pour la désorption de CO₂ gazeux, H₂O et SO₂ gazeux est de 6 à 10 minutes.

8. Procédé selon la revendication 7, **caractérisé en ce que** le traitement de température est de 6 à 8 minutes.

9. Procédé selon l'une quelconque des revendications 1 à 8, **caractérisé en ce que** la mesure de température est réalisée directement sur le chauffage.

10. Procédé selon l'une quelconque des revendications 1 à 9, **caractérisé en ce qu'**entre des mesures individuelles d'un échantillon, la structure est refroidie de manière forcée à température ambiante.

11. Procédé selon l'une quelconque des revendications 1 à 10, **caractérisé en ce que** le courant de volume gazeux est de 200 ml/min à 700 ml/min.

12. Procédé selon l'une quelconque des revendications 1 à 11, **caractérisé en ce que** des quantités d'échantillons supérieures à 10 mg peuvent être séparées et analysées.

13. Procédé selon l'une quelconque des revendications 1 à 12, **caractérisé en ce que** les modifications correspondantes entre les températures de désorption sont réalisées comme une fonction à échelons.

14. Procédé selon l'une quelconque des revendications 1 à 12, **caractérisé en ce que** les modifications peuvent également être réalisées de manière linéaire entre les températures de désorption.

15. Dispositif installé pour la réalisation du procédé selon l'une quelconque des revendications 1 à 14 avec une colonne d'adsorption individuelle pouvant être chauffée par une installation de chauffage, laquelle est remplie d'un matériau adsorbant, la colonne d'adsorption présentant une entrée de gaz pour l'introduction du courant de gaz principal ainsi qu'une sortie de gaz.

16. Dispositif selon la revendication 15, **caractérisé en ce que** l'installation de chauffage est appliquée directement sur le côté externe de la colonne d'adsorption.

17. Dispositif selon la revendication 15 ou 16, **caractérisé en ce que** la colonne d'adsorption est formée par un tuyau avec de bonnes caractéristiques de conduction de chaleur et une surface résistante à la corrosion.

18. Dispositif selon la revendication 17, **caractérisé en ce que** le tuyau est constitué d'acier inoxydable.

19. Dispositif selon la revendication 17, **caractérisé en ce que** le tuyau d'adsorption présente une longueur d'environ 250 mm à 350 mm, de préférence de 300 mm.

20. Dispositif selon la revendication 19, **caractérisé en ce que** le diamètre interne du tuyau d'adsorption est de 4 mm à 10 mm, de préférence d'environ 6 mm.

21. Dispositif selon l'une quelconque des revendications 17 à 20, **caractérisé en ce que** le tuyau d'adsorption est courbé en forme de U.

22. Dispositif selon l'une quelconque des revendications 15 à 21, **caractérisé en ce que** le tuyau d'adsorption est ajouté à une installation de refroidissement forcé.

23. Dispositif selon la revendication 22, **caractérisé en ce que** l'installation de refroidissement forcé est formée par une soufflante d'air.

24. Dispositif selon l'une quelconque des revendications 15 à 23, **caractérisé en ce que** la colonne d'adsorption est remplie d'un tamis moléculaire de charbon comme matériau d'adsorption.

25. Dispositif selon l'une quelconque des revendications 15 à 24, **caractérisé en ce qu'**un thermoélément est appliqué sur l'installation du chauffage.
